**Europäisches Patentamt**

**European Patent Office**

**Office européen des brevets**

(11) Veröffentlichungsnummer : **0 380 911 B1**

# (12) EUROPÄISCHE PATENTSCHRIFT

(45) Veröffentlichungstag der Patentschrift :
**27.12.91 Patentblatt 91/52**

(21) Anmeldenummer : **90100128.9**

(22) Anmeldetag : **04.01.90**

(51) Int. Cl.⁵ : **B01J 31/40**, C07C 51/42, C07C 53/08, C07C 51/373, C07C 53/12, B01J 38/04

---

(54) **Verfahren zur Reinigung und Rückgewinnung der bei der Carbonylierung von Methanol und/oder Methylacetat und/oder Dimethylether anfallenden verunreinigten Katalysatorlösung.**

---

(30) Priorität : **28.01.89 DE 3902515**

(43) Veröffentlichungstag der Anmeldung :
**08.08.90 Patentblatt 90/32**

(45) Bekanntmachung des Hinweises auf die
Patenterteilung :
**27.12.91 Patentblatt 91/52**

(84) Benannte Vertragsstaaten :
**AT BE CH DE FR GB IT LI NL**

(56) Entgegenhaltungen :
**EP-A- 0 073 341**
**DE-A- 2 533 320**
**GB-A- 2 054 394**

(73) Patentinhaber : **HOECHST
AKTIENGESELLSCHAFT
Postfach 80 03 20
W-6230 Frankfurt am Main 80 (DE)**

(72) Erfinder : **Erpenbach, Heinz, Dr.
Oberbuschweg 22
W-5000 Köln (DE)**
Erfinder : **Goedicke, Eitel, Dr.
Asternweg 7
W-5100 Bergheim (DE)**
Erfinder : **Lork, Winfried
Siegfried-von-Westerburg-Strasse 14
W-5042 Erftstadt (DE)**
Erfinder : **Tetzlaff, Heribert, Dr.
Geisenheimer Strasse 88
W-6000 Frankfurt (DE)**

---

**Beschreibung**

Die Erfindung betrifft ein Verfahren zur Reinigung und Rückgewinnung der bei der Carbonylierung von Methanol und/oder Methylacetat und/oder Dimethylether anfallenden verunreinigten Katalysatorlösung, welche Carbonylkomplexe des Rhodiums, quaternäre heterocyclische aromatische Stickstoffverbindungen oder quaternäre Organophosphorverbindungen als organische Promotoren und/oder Alkalimetallsalze und ggf. Verbindungen carbonylbildender Nichtedelmetalle als anorganische Promotoren, undestillierbare organische Verunreinigungen sowie Essigsäure, Essigsäureanhydrid und Ethylidendiacetat enthält.

Zur Durchführung von Hydroformylierungs- und Carbonylierungsverfahren wird als Edelmetallkatalysator Rhodium in Form vielfältiger komplexer Verbindungen eingesetzt. Da Rhodium aufgrund seiner geringen Verfügbarkeit ein sehr teures Edelmetall ist, wird über seine Rückgewinnung bzw. die Reinigung von Rhodiumkomplexen aus mit Rückständen verunreinigten Katalysatorsystemen bzw. Destillationssümpfen der oben angeführten Reaktionen mehrfach in der Literatur berichtet.

Die EP-A-0240703 (= US-A-4,746,640) beansprucht ein Verfahren, das es gestattet, die bei der Carbonylierung von Methylacetat und/oder Dimethylether anfallende, verunreinigte rhodiumhaltige Katalysatorlösung so zu reinigen, daß die bei der Umsetzung gebildeten undestillierbaren organischen Rückstände durch eine Flüssig-Flüssig-Extraktion mit Dialkylethern und Alkanolen mit jeweils 1-4 C-Atomen der Lösung entzogen werden. Die die organischen Rückstände enthaltende Etherphase wird einer Nachbehandlung mit Jod und/oder Methyljodid unterzogen, vom ausgefallenen restlichen Katalysatorkomplex abgetrennt und dann weiter destillativ in wiederzuverwendenden Dialkylether und wiederzuverwendendes Alkanol Essigsäure, Essigsäureanhydrid, Ethylidendiacetat und undestillierbaren Rückstand getrennt. Essigsäure, Essigsäureanhydrid und Ethylidendiacetat werden mit der gereinigten Katalysatorphase sowie dem aus der Etherphase isolierten Katalysatorkomplex vereinigt, vom restlichen Ether und Alkohol befreit und als frische Katalysatorlösung der Reaktion zugeführt. In dem ausgeschleusten Rückstand der Etherphasendestillation verbleiben nur noch < 0,1% des in die Extraktion eingesetzten Rhodiums.

Der hohe Preis des Rhodiums gestattet nur seine verlustfreie Verwendung als Katalysator für großtechnische Verfahren. Dies gilt auch für die Reinigung und Rückgewinnung eines eingesetzten verunreinigten Rhodiumkatalysators und macht daher einen quantitativen Kreislauf unerläßlich. Dies erfordert eine ohne Zwischenstufen verlaufende, möglichst einfache Abtrennung des im Verfahren gebildeten organischen Rückstandes aus der Katalysatorlösung ohne jeden Rh-Verlust und den direkten Wiedereinsatz des gereinigten Katalysatorsystems in die Reaktion.

Das Verfahren der EP-A-0240703 (= US-A-4,746,640) erfüllt die aufgestellten Forderungen voll und weist mit 99,9% eine sehr gute Rhodiumrückgewinnungsrate auf, ist jedoch im Trennungsgang äußerst kostenaufwendig.

Die GB-A-2054394 beansprucht ein Verfahren zur Rückgewinnung von Katalysatoren, einschließlich Edelmetall-Koordinationsverbindungen, aus einem in einem homogenen katalytischen Reaktionssystem gebildeten Reaktionsgemisch, worin man das Reaktionssystem mit einem verflüssigten Gas mit einer kritischen Temperatur im Bereich von 0 bis 100°C, das gegenüber dem Reaktionssystem inert ist, in einer Extrahiervorrichtung bei einer Temperatur über seiner kritischen Temperatur und einem Druck über seinem kritischen Druck in Kontakt bringt. Die gleichzeitige Rückgewinnung der in Anspruch 1 vorliegender Erfindung beschriebenen organischen Promotoren ist nicht naheliegend.

Die vorliegende Erfindung beschreibt ein Verfahren, das das überraschend hohe Extraktionsvermögen verflüssigter oder überkritischer Gase für die Reinigung der verunreinigten Katalysatorlösung von organischen Verunreinigungen bzw. Rückständen ausnutzt. So ist eine einfache Reinigung der bei der Carbonylierung von Methanol und/oder Methylacetat und/oder Dimethylether ein- gesetzten und im Laufe des Prozesses verunreinigten Katalysatorlösung durch Extraktion bevorzugt mit dem physiologisch unbedenklichen $CO_2$ (Kohlendioxid) möglich, wobei die Abtrennung der undestillierbaren organischen Verunreinigungen aus der Katalysatorlösung ohne Rhodiumverlust und ohne Zerstörung des Katalysatorkomplexes sowie des Promotors erfolgt. Rh-Komplex und Promotorsalz können ohne zusätzliche Maßnahmen dem Carbonylierungsprozeß wieder zugeführt werden. Durch den Kreislaufbetrieb des zur Aufarbeitung eingesetzten inerten Extraktionsmittels $CO_2$ wird eine zusätzliche Umweltbelastung durch Abfallstoffe vermieden. Nur die im Prozeß gebildeten organischen Verunreinigungen werden ausgeschleust und können entsprechend dem Stand der Technik beseitigt werden. Der Extraktionsprozeß des Verfahrens der Erfindung wird je nach Druck- und Temperaturbedingungen in Form einer Hochdruck-Flüssig-Flüssig-Extraktion oder einer Hochdruckextraktion mit überkritischen Gasen durchgeführt. Eine kontinuierliche Arbeitsweise ist in jeder druck- und temperatursteuerbaren Extraktionsapparatur technisch realisierbar. Dabei erlaubt die Extraktion in z.B. einer Gegenstromkolonne die Minimierung der Extraktionsmittelmenge.

Im einzelnen ist das Verfahren der Erfindung dadurch gekennzeichnet, daß man der verunreinigten Katalysatorlösung durch Extraktion mit einem verflüssigten oder überkritischen Gas, ausgewählt aus der Gruppe Kohlendioxid, Schwefelhexafluorid, Distickstoffmonoxid, Fluorkohlenwasserstoffe, Chlorfluorkohlenwasserstoffe, Bromfluorkohlenwasserstoffe, Bromchlorfluorkohlenwasserstoffe, gesättigte oder ungesättigte $C_2$-$C_4$-Kohlenwasserstoffe, Ammoniak oder deren Gemische bei Drucken von 35 bis 450 bar und Temperaturen von 0 bis 120°C die organischen Verunreinigungen sowie Essigsäure, Essigsäureanhydrid und Ethylidendiacetat entzieht und die Phase des verflüssigten oder überkritischen Gases von der gereinigten promotorhaltigen Katalysatorlösung abtrennt ; daß man die Phase des verflüssigten oder überkritischen Gases durch Entspannungsverdampfung auftrennt, das zurückgewonnene Gas erneut zur Extraktion einsetzt, aus dem flüssig verbleibenden Anteil Essigsäure, Essigsäureanhydrid und Ethylidendiacetat abtrennt und mit dem gereinigten Katalysatorkomplex zur Bereitung einer frischen Katalysatorlösung vereinigt ; und daß man die bei der Auftrennung der Phase des verflüssigten oder überkritischen Gases als Rückstand verbleibenden organischen Verunreinigungen ausschleust.

Das Verfahren der Erfindung kann weiterhin bevorzugt und wahlweise dadurch gekennzeichnet sein, daß

a) man je Gewichtsteil verunreinigter Katalysatorlösung 0,5 bis 80, vorzugsweise 5 bis 20, Gewichtsteile verflüssigtes oder überkritisches Gas, vorzugsweise $CO_2$, einsetzt ;

b) man je Gewichtsteil verunreinigter Katalysatorlösung zusätzlich 0,03 bis 0,4 Gewichtsteile Essigsäure und/oder Essigsäureanhydrid und-/oder Ethylidendiacetat zusetzt ;

c) man je Gewichtsteil verunreinigter Katalysatorlösung 0,03 bis 0,4 Gewichtsteile Methanol zusetzt ;

d) die Temperatur zwischen der Verflüssigungs- oder der kritischen Temperatur des Gases und 120°C liegt ;

e) der Druck zwischen dem Verflüssigungs- oder dem kritischen Druck des Gases und 450 bar liegt.

Die aus einem Carbonylierungsreaktor abfließende Reaktionsmischung wird destillativ in die gewünschten Endprodukte, insbesondere Essigsäureanhydrid und Essigsäure, sowie nichtumgesetzte, im Kreislauf geführte Ausgangsstoffe einerseits und die als Sumpfprodukt anfallende und im Kreislauf geführte Katalysatorlösung andererseits aufgetrennt. Ein Teilstrom dieser mit der Zeit durch undestillierbare organische Produkte verunreinigten Katalysatorlösung, welche je nach Verfahrensbedingungen bis zu 75 M-% (= Masse-%) an Essigsäureanhydrid, Essigsäure und/oder Ethylidendiacetat enthalten kann, wird aus dem Kreislauf der Katalysator lösung entnommen und der Reinigung zugeführt. Die Katalysatorlösung enthält das Edelmetall Rhodium als Carbonylkomplex wie z.B.
$[CH_3P(C_4H_9)_3]$ $Rh(CO)I_4$ ; $[CH_3P(C_4H_9)_3]$ $Rh(CO)_2I_2$ oder $[C_5H_9N_2]$ $Rh(CO)_2I_2$.

Die Katalysatorlösung kann ferner als organische Promotoren bevorzugt einen oder mehrere der folgenden heterocyclischen aromatischen Stickstoffverbindungen oder Organophosphorverbindungen enthalten :

1. N-Methylpyridiniumiodid, N,N-Dimethylimidazoliumiodid, N-Methyl-3-picoliniumiodid, N-Methyl-2,4-lutidiniumiodid, N-Methyl-3,4-lutidiniumiodid, N-Methyl-chinoliniumiodid ;

2. Tri-n-butyl-methyl-phosphoniumiodid, Trioctylmethylphosphoniumiodid, Trilauryl-methyl-phosphoniumiodid, Triphenylmethyl-phosphoniumiodid.

Schließlich kann die Katalysatorlösung als anorganische Promotoren Alkalimetallsalze wie z.B. Lithiumiodid, Lithiumacetat, Kaliumiodid oder Natriumiodid und Verbindungen der carbonylbildenden Nichtedelmetalle Ce, Ti, Zr, Hf, Ge, Sn, Pb, V, Nb, Ta, As, Sb, Bi, Cr, Mo, W, Mn, Re, Fe, Co, Ni enthalten.

Die ausgeschleuste, verunreinigte Katalysatorlösung wird mit dem verflüssigten oder überkritischen Gas, vorzugsweise $CO_2$, bei 0 bis 120°C (35 bis 450 bar) extrahiert. Hierbei werden die bei der Reaktion gebildeten undestillierbaren organischen Verunreinigungen sowie die in der Katalysatorlösung befindlichen Anteile Essigsäureanhydrid, Essigsäure und/oder Ethylidendiacetat extrahiert, während der Rh-Carbonylkomplex mit dem oder den Promotoren als Katalysatorphase zurückbleibt. Aus der $CO_2$-haltigen Phase wird das $CO_2$ nach Entspannung zurückgewonnen und nach Kompression wieder in die Extraktion zurückgeführt. Anschließend werden Essigsäureanhydrid, Essigsäure und/oder Ethylidendiacetat redestilliert, wobei die undestillierbaren organischen Verunreinigungen als Rückstand anfallen. Die redestillierten Reaktionsprodukte werden der gereinigten Katalysatorphase (Rh-Carbonylkomplex und Promotorsalz) zugesetzt und in den Carbonylierungsprozeß zurückgeführt. Die undestillierbaren organischen Verunreinigungen werden z.B. in einer Verbrennungsanlage vernichtet.

Das Verfahren der Erfindung kann in sowohl kontinuierlicher als auch diskontinuierlicher Betriebsweise durchgeführt werden.

Beispiel 1

Zur Entfernung der organischen Verunreinigungen werden dem Katalysatorkreislauf der Methanol/Methylacetatcarbonylierung 400 g Katalysatorlösung der Zusammensetzung 6,1 M.-% Rhodiumcarbonylkomplex $[CH_3P(C_4H_9)_3]$ $[Rh(CO)_2I_2]$ ($\hat{=}$ 4,0 g = 1,0 M.-% Rh), 67,5 M.-% Methyl-tri-n-butylphosphoniumiodid, 2,75 M.-% organische Verunreinigungen und 23,65 M.-% eines Gemisches aus Essigsäure, Essigsäureanhydrid und Ethylidendiacetat entnommen und mit 2.900 g $CO_2$ bei 80°C und 300 bar extrahiert. Die $CO_2$-Phase wird von der Katalysatorphase abgetrennt und nach Entspannen auf einen Druck von 50 bar bei 16°C in $CO_2$ und 104,5 g Extrakt aufgetrennt. Das $CO_2$ wird nach Komprimieren und Temperatureinstellung auf Extraktionsbedingungen zur erneuten Extraktion eingesetzt, während der Extrakt in 94,6 g Essigsäure-Essigsäureanhydrid-Ethylidendiacetat-Gemisch und 9,9 g undestillierbare organische Verunreinigungen als teerartiger Rückstand (Rh-Gehalt 0,04 M.-%) destillativ zerlegt wird. Das gewonnene Gemisch aus Essigsäure, Essigsäureanhydrid und Ethylidendiacetat wird mit der raffinierten Katalysatorphase vereinigt und als 390,1 g gereinigte Katalysatorlösung mit einem Rh-Gehalt von 4 g dem Katalysatorkreislauf wieder zugesetzt. Die Rückführungsrate des Rhodiums in den Carbonylierungsprozeß beträgt nach der Reinigung der abgezogenen verunreinigten Katalysatorlösung 99,90%.

## Beispiel 2

Zur Entfernung der organischen Verunreinigungen werden dem Katalysatorkreislauf der Methanol/Methylacetatcarbonylierung 400 g Katalysatorlösung der Zusammensetzung 4,9 M.-% Rhodiumcarbonylkomplex [Li] [Rh(CO)$_2$I$_2$] ($\hat{=}$ 4,8 g = 1,2 M.-% Rh), 49,1 M.-% Lithiumiodid, 4,0 M.-% organische Verunreinigungen und 42,0 M.-% eines Gemisches aus Essigsäure, Essigsäureanhydrid und Ethylidendiacetat entnommen und mit 3.200 g $CO_2$ bei 40°C und 100 bar extrahiert. Die $CO_2$-Phase wird von der Katalysatorphase abgetrennt und nach dem Entspannen auf einen Druck von 50 bar bei 16°C in $CO_2$ und 182,1 g Extrakt aufgetrennt. Das $CO_2$ wird nach Komprimieren und Temperatureinstellung auf Extraktionsbedingungen zur erneuten Extraktion eingesetzt, während der Extrakt in 168 g Essigsäure-Essigsäureanhydrid-Ethylidendiacetat-Gemisch und 14,1 g undestillierbare organische Verunreinigungen als teerartiger Rückstand (Rh-Gehalt 0,03 M.-%) destillativ zerlegt wird. Das gewonnene Gemisch aus Essigsäure, Essigsäureanhydrid und Ethylidendiacetat wird mit der raffinierten Katalysatorphase vereinigt und als 385,9 g gereinigte Katalysatorlösung mit einem Rh-Gehalt von 4,8 g dem Katalysatorkreislauf wieder zugesetzt. Die Rückführungsrate des Rhodiums in den Carbonylierungsprozeß beträgt nach der Reinigung der abgezogenen verunreinigten Katalysatorlösung 99,91%.

## Beispiel 3

Zur Entfernung der organischen Verunreinigungen werden dem Katalysatorkreislauf der Dimethylethercarbonylierung 400 g Katalysatorlösung der Zusammensetzung 6,05 M.-% Rhodiumcarbonylkomplex [C$_5$H$_9$N$_2$] [Rh(CO)$_2$I$_2$] ($\hat{=}$ 4,88 g =1,22 M.-% Rh), 45,35 M.-% N,N-Dimethylimidazoliumjodid, 3,5 M.-% organische Verunreinigungen und 45,1 M.-% eines Gemisches aus Essigsäure, Essigsäureanhydrid und Ethylidendiacetat entnommen und mit 2.800 g $CO_2$ unter Zusatz von 300 g eines Gemisches aus Essigsäure, Essigsäureanhydrid und Ethylidendiacetat bei 60°C und 150 bar extrahiert. Die $CO_2$-Phase wird von der Katalysatorphase abgetrennt und nach dem Entspannen auf einen Druck von 35 bar bei 16°C in $CO_2$ und 311,5 g Extrakt aufgetrennt. Das $CO_2$ wird nach Komprimieren und Temperatureinstellung auf die Extraktionsbedingungen zur erneuten Extraktion eingesetzt, während der Extrakt in 300 g Essigsäure-Essigsäureanhydrid-Ethylidendiacetat-Gemisch und 11,5 g undestillierbare organische Verunreinigungen als teerartiger Rückstand (Rh-Gehalt 0,03 M.-%) destillativ zerlegt wird. Das gewonnene Gemisch aus Essigsäure, Essigsäureanhydrid und Ethylidendiacetat wird zur erneuten Extraktion verwendet, während das Raffinat als 388,5 g gereinigte Katalysatorlösung mit einem Rh-Gehalt von 4,88 g dem Katalysatorkreislauf wieder zugesetzt wird. Die Rückführungsrate des Rhodiums in den Carbonylierungsprozeß beträgt nach der Reinigung der abgezogenen verunreinigten Katalysatorlösung 99,93%.

## Beispiel 4

Zur Entfernung der organischen Verunreinigungen werden dem Katalysatorkreislauf der Methanol/Methylacetatcarbonylierung 400 g Katalysatorlösung der Zusammensetzung 3,99 M.-Rhodiumcarbonylkomplex [CH$_3$P(C$_4$H$_9$)$_3$][Rh(CO)I$_4$] ($\hat{=}$ 1,92 g = 0,48 M.-% Rh), 64,9 M.-% Methyl-tri-n-butylphosphoniumiodid, 3,2 M.-% organische Verunreinigungen und 27, 91 M.-% eines Gemisches aus Essigsäure, Essigsäureanhydrid und Ethylidendiacetat entnommen und mit 2500 g $CO_2$ unter Zusatz von 250 g Methanol bei 25°C und 70 bar extrahiert. Die $CO_2$-Phase wird von der Katalysatorphase abgetrennt und nach dem Entspannen auf einen Druck von 50 bar bei 25°C in $CO_2$ und 260,4 g Extrakt aufgetrennt. Das $CO_2$ wird nach Komprimieren und Temperatureinstellung auf die Extraktionsbedingungen zur erneuten Extraktion eingesetzt, während der Extrakt in 250,0 g Essigsäure-Essigsäureanhydrid-Ethylidendiacetat-Methanol-Gemisch und 10,4 g undestillierbare organische Verunreinigungen als teerartiger Rückstand (Rh-Gehalt 0,015 M.-%) destillativ zerlegt wird. Das gewonnene Gemisch aus Essigsäure, Essigsäureanhydrid, Ethylidendiacetat und Methanol wird mit der raffinierten Katalysatorphase vereinigt und als 639,6 g gereinigte Katalysatorlösung mit einem Rh-Gehalt von 1,92 g dem Katalysatorkreislauf wieder zugesetzt. Die Rückführungsrate des Rhodiums in den Carbonylierungsprozeß beträgt nach der Reinigung der abgezogenen verunreinigten Katalysatorlösung 99,92%. Das bei der Extraktion eingesetzte Methanol braucht nicht aus der Katalysatorlösung entfernt zu werden, da es im Carbonylierungsreaktor zu den gewünschten Reaktionsprodukten Essigsäure und Essigsäureanhydrid umgesetzt wird.

Beispiel 5

Zur Entfernung der organischen Verunreinigungen werden dem Katalysatorkreislauf der Dimethylethercarbonylierung 400 g Katalysatorlösung der Zusammensetzung 6,05 M.-% Rhodiumcarbonylkomplex $[C_5H_9N_2]$ $[Rh(CO)_2I_2]$ ($\hat{=}$ 4,88 g = 1,22 M.-% Rh), 45,35 M.-% N,N-Dimethylimidazoliumjodid, 3,5 M.-% organische Verunreinigungen und 45,1 M.-% eines Gemisches aus Essigsäure, Essigsäureanhydrid und Ethylidendiacetat entnommen und mit 4000 g Ethylen unter Zusatz von 300 g eines Gemisches aus Essigsäure, Essigsäureanhydrid und Ethylidendiacetat bei 40°C und 300 bar extrahiert. Die Ethylenphase wird von der Katalysatorphase abgetrennt und nach dem Entspannen auf einen Druck von 50 bar bei 5°C in Ethylen und 311,6 g Extrakt aufgetrennt. Das Ethylen wird nach Komprimieren und Temperatureinstellung auf die Extraktionsbedingungen zur erneuten Extraktion eingesetzt, während der Extrakt in 300 g Essigsäure-Essigsäureanhydrid-Ethyliden diacetat-Gemisch und 11,6 g undestillierbare organische Verunreinigungen als teerartiger Rückstand (Rh-Gehalt 0,02 M.-%) destillativ zerlegt wird. Das gewonnene Gemisch aus Essigsäure, Essigsäureanhydrid und Ethylidendiacetat wird zur erneuten Extraktion verwendet, während das Raffinat als 388,4 g gereinigte Katalysatorlösung mit einem Rh-Gehalt von 4,88 g dem Katalysatorkreislauf wieder zugesetzt wird. Rhodium-Rückführungsrate : 99,95%.

Beispiel 6

Zur Entfernung der organischen Verunreinigungen werden dem Katalysatorkreislauf der Methanol/Methylacetatcarbonylierung 400 g Katalysatorlösung der Zusammensetzung 3,99 M.-% Rhodiumcarbonylkomplex $[CH_3P(C_4H_9)_3]$ $[Rh(CO)I_4]$ ($\hat{=}$ 1,92 g = 0,48 M.-% Rh), 64,9 M.-% Methyl-tri-n-butylphosphoniumiodid, 3,2 M.-% organische Verunreinigungen und 27,91 M.-% eines Gemisches aus Essigsäure, Essigsäureanhydrid und Ethylidendiacetat entnommen und mit 2500 g ® Frigen 23 ($CHF_3$) unter Zusatz von 300 g Methanol bei 50°C und 150 bar extrahiert. Die ® Frigen 23-Phase wird von der Katalysatorphase abgetrennt und nach dem Entspannen bei 1 bar und 19°C in ® Frigen 23 und 310,2 g Extrakt aufgetrennt. Das ® Frigen 23 wird nach Komprimieren und Temperatureinstellung auf die Extraktionsbedingungen zur erneuten Extraktion eingesetzt, während der Extrakt in 300 g Essigsäure-Essigsäureanhydrid-Ethylidendiacetat-Methanol-Gemisch und 10,2 g undestillierbare organische Verunreinigungen als teerartiger Rückstand (Rh-Gehalt 0,018 M.-%) destillativ zerlegt wird. Das gewonnene Gemisch aus Essigsäure, Essigsäureanhydrid, Ethylidendiacetat und Methanol wird mit der raffinierten Katalysatorphase vereinigt und als 689,8 g gereinigte Lösung mit einem Rh-Gehalt von 1,92 g dem Katalysatorkreislauf wieder zugesetzt. Rhodium-Rückführungsrate : 99,90%.

## Patentansprüche

1. Verfahren zur Reinigung und Rückgewinnung der bei der Carbonylierung von Methanol und/oder Methylacetat und/oder Dimethylether anfallenden verunreinigten Katalysatorlösung, welche Carbonylkomplexe des Rhodiums, quaternäre heterocyclische aromatische Stickstoffverbindungen oder quaternäre Organophosphorverbindungen als organische Promotoren, und/oder Alkalimetallsalze und ggf. Verbindungen carbonylbildender Nichtedelmetalle als anorganische Promotoren, undestillierbare organische Verunreinigungen sowie Essigsäure, Essigsäureanhydrid und Ethylidendiacetat enthält, dadurch gekennzeichnet, daß man der verunreinigten Katalysatorlösung durch Extraktion mit einem verflüssigten oder überkritischen Gas, ausgewählt aus der Gruppe Kohlendioxid, Schwefelhexafluorid, Distickstoffmonoxid, Fluorkohlenwasserstoffe, Chlorfluorkohlenwasserstoffe, Bromfluorkohlenwasserstoffe, Bromchlorfluorkohlenwasserstoffe, gesättigte oder ungesättigte $C_2$- bis $C_4$ Kohlenwasserstoffe, Ammoniak oder deren Gemische, bei Drucken von 35 bis 450 bar und Temperaturen von 0 bis 120°C die organischen Verunreinigungen sowie Essigsäure, Essigsäureanhydrid und Ethylidendiacetat entzieht und die Phase des verflüssigten oder überkritischen Gases von der gereinigten promotorhaltigen Katalysatorlösung abtrennt ; daß man die Phase des verflüssigten oder überkritischen Gases durch Entspannungsverdampfung auftrennt, das zurückgewonnene Gas erneut zur Extraktion einsetzt, aus dem flüssig verbleibenden Anteil Essigsäure, Essigsäureanhydrid und Ethylidendiacetat abtrennt und mit dem gereinigten Katalysatorkomplex zur Bereitung einer frischen Katalysatorlösung vereinigt ; und daß man die bei der Auftrennung der Phase des verflüssigten oder überkritischen Gases als Rückstand verbleibenden organischen Verunreinigungen ausschleust.

2. Verfahren nach Anspruch 1, dadurch gekennzeichnet, daß man je Gewichtsteil verunreinigter Katalysatorlösung 0,5 bis 80, vorzugsweise 5 bis 20, Gewichtsteile verflüssigtes oder überkritisches Gas, vorzugsweise $CO_2$, einsetzt.

3. Verfahren nach mindestens einem der vorher-

gehenden Ansprüche, <u>dadurch gekennzeichnet</u>, daß man je Gewichtsteil verunreinigter Katalysatorlösung zusätzlich 0,03 bis 0,4 Gewichtsteile Essigsäure und-/oder Essigsäureanhydrid und/oder Ethylidendiacetat zusetzt.

4. Verfahren nach mindestens einem der vorhergehenden Ansprüche, <u>dadurch gekennzeichnet</u>, daß man je Gewichtsteil verunreinigter Katalysatorlösung 0,03 bis 0,4 Gewichtsteile Methanol zusetzt.

5. Verfahren nach mindestens einem der vorhergehenden Ansprüche, <u>dadurch gekennzeichnet</u>, daß die Temperatur zwischen der Verflüssigungs- oder der kritischen Temperatur des Gases und 120°C liegt.

6. Verfahren nach mindestens einem der vorhergehenden Ansprüche, <u>dadurch gekennzeichnet</u>, daß der Druck zwischen dem Verflüssigungs- oder dem kritischen Druck des Gases und 450 bar liegt.


## Claims

1. A process for the purification and recovery of the contaminated solution of catalyst produced on carbonylation of methanol and/or methyl acetate and/or dimethyl ether and containing carbonyl complexes of rhodium, quaternary heterocyclic aromatic nitrogen compounds or quaternary organo-phosphorus compounds as organic promoters, and/or alkali metal salts and, if desired, compounds of carbonyl-forming base metals as inorganic promoters, undistillable organic impurities and acetic acid, acetic anhydride and ethylidene diacetate, which comprises removing the organic impurities as well as acetic acid, acetic anhydride and ethylidene diacetate from the contaminated solution of catalyst by extraction at pressures from 35 to 450 bar and temperatures from 0 to 120°C with a liquefied or supercritical gas selected from the group comprising carbon dioxide, sulfur hexafluoride, dinitrogen monoxide, fluorohydrocarbons, chlorofluorohydrocarbons, bromofluorohydrocarbons, bromochlorofluorohydrocarbons, saturated or unsaturated $C_2$- to $C_4$-hydrocarbons, ammonia or mixtures thereof, and separating the phase of the liquefied or supercritical gas from the purified promoter-containing catalyst solution, separating the phase of the liquefied or supercritical gas by flash vaporization, re-employing the recovered gas for extraction, separating acetic acid, acetic anhydride and ethylidene diacetate from the component which remains liquid and combining it with the purified catalyst complex for preparing fresh catalyst solution, and purging the organic impurities which remain as the residue when the phase of the liquefied or supercritical gas is separated.

2. The process as claimed in claim 1, wherein 0.5 to 80, preferably 5 to 20, parts by weight of liquefied or supercritical gas, preferably $CO_2$, are employed per part by weight of contaminated catalyst solution.

3. The process as claimed in at least one of the preceding claims, wherein additionally 0.03 to 0.4 part by weight of acetic acid and/or acetic anhydride and/or ethylidene diacetate is added per part by weight of contaminated catalyst solution.

4. The process as claimed in at least one of the preceding claims, wherein 0. 03 to 0. 4 part by weight of methanol is added per part by weight of contaminated catalyst solution.

5. The process as claimed in at least one of the preceding claims, wherein the temperature is between the liquefaction temperature or critical temperature of the gas and 120°C.

6. The process as claimed in at least one of the preceding claims, wherein the pressure is between the liquefaction pressure or critical pressure of the gas and 450 bar.


## Revendications

1. Procédé pour la purification et la récupération de la solution de catalyseur contaminée se formant dans la carbonylation du méthanol et/ou de l'acétate de méthyle et/ou de l'oxyde de diméthyle, qui contient des complexes de carbonyle du rhodium, des composés azotés aromatiques hétérocycliques ou des composés organo-phosphorés quaternaires comme promoteurs organiques et/ou des sels de métaux alcalins et éventuellement des composés de métaux non nobles formant des complexes de carbonyle comme promoteurs inorganiques, des impuretés organiques non distillables ainsi que de l'acide acétique, de l'anhydride acétique et du diacétate d'éthylidène, caractérisé en ce que l'on retire de la solution de catalyseur contaminée les impuretés organiques ainsi que l'acide acétique, l'anhydride acétique et le diacétate d'éthylidène par extraction par un gaz liquéfié ou surcritique, choisi parmi le dioxyde de carbone, l'hexafluorure de soufre, le protoxyde d'azote, les hydrocarbures fluorés, les hydrocarbures chlorofluorés, les hydrocarbures bromofluorés, les hydrocarbures bromochlorofluorés, les hydrocarbures saturés ou insaturés en $C_2$-$C_4$, l'ammoniac ou leurs mélanges, sous des pressions de 35 a 450 bar et à des températures de 0 a 120°C et on sépare la phase du gaz liquéfié ou surcritique de la solution purifiée de catalyseur contenant le promoteur ; en ce que l'on sépare la phase du gaz liquéfié ou surcritique par évaporation par détente, on réutilise le gaz récupéré pour l'extraction, on sépare de la fraction restant liquide l'acide acétique, l'anhydride acétique et le diacétate d'éthylidène et on les réunit avec le complexe catalyseur purifié pour la préparation d'une solution fraîche de catalyseur ; et en ce que l'on évacue les impuretés organiques restant comme résidu dans la séparation de la phase du gaz liquéfié ou surcritique.

2. Procédé selon la revendication 1, caractérisé en ce que l'on utilise par partie en poids de solution contaminée de catalyseur 0,5 à 80, de préférence 5 à 20, parties en poids de gaz liquéfié ou surcritique, de préférence $CO_2$.

3. Procédé selon l'une au moins des revendications précédentes, caractérisé en ce que l'on ajoute en outre par partie en poids de solution contaminée de catalyseur 0,03 à 0,4 partie en poids d'acide acétique et/ou d'anhydride acétique et/ou de diacétate d'éthylidène.

4. Procédé selon l'une au moins des revendications précédentes, caractérisé en ce que l'on ajoute par partie en poids de solution contaminée de catalyseur 0,03 à 0,4 partie en poids de méthanol.

5. Procédé selon l'une au moins des revendications précédentes, caractérisé en ce que la température est comprise entre la température de liquéfaction ou la température critique du gaz et 120°C.

6. Procédé selon l'une au moins des revendications précédentes, caractérisé en ce que la pression est comprise entre la pression de liquéfaction ou la pression critique du gaz et 450 bar.